# EUROPEAN PATENT APPLICATION

(11) **EP 3 882 338 A1**
(43) Date of publication of application: **22.09.2021**
(21) Application number: 19876188.4
(22) Date of filing: 25.10.2019
(51) Int. Cl.: C12N 5/071, C12N 7/00, G01N 33/50, A01K 67/027

(54) **METHOD FOR PREPARING VIRUS-INFECTED CELL LINE AND ANIMAL MODEL**

(30) Priority: 25.10.2018 KR 20180128199; 25.10.2018 KR 20180128201
(71) Applicant: Industry-Academic Cooperation Foundation, Yonsei University, Seoul 03722 (KR); University Industry Foundation, Yonsei University Wonju Campus, Gangwon-do 26493 (KR)
(72) Inventor: LEE, Hong Jai, Seoul 05501 (KR); KIM, Soo Ki, Seoul 05649 (KR); MOON, Jae Seung, Seoul 04356 (KR); SHIN, Bo Young, Daegu 42766 (KR); LEE, Sang Kyou, Seoul 06002 (KR); SHIN, Jin Su, Seoul 03725 (KR)
(74) Representative: Schnappauf, Georg
(86) International application number: PCT/KR2019/014153
(87) International publication number: WO 2020/085846

(57) **Abstract**

The present invention relates to a method for producing virus-infected cell line or animal model. According to the present invention, for a human-derived lipid layer of a virus, a bile acid or a bile acid derivative allows the lipid layer to be replaced with a target animal model-derived lipid layer; and this makes it possible to produce a virus-infected animal model that can be effectively used clinically without limitation resulting from species specificity. Furthermore, in the method for producing a cell line, viruses are mixed with a bile acid or a bile acid derivative and the mixture is used to treat a culture medium of a cell line, so that a cell line infected with a very high level of the viruses can be obtained. In addition, use of the method for producing a cell line of the present invention allows viruses to infect cell lines derived from various species and organs without limitation resulting from the viral species specificity and the restraining force related to the viral specific tissue or organ tropism.

## Description

### Technical Field

The present invention relates to a method for producing virus-infected cell line or animal model, and more specifically, to a method for producing virus-infected cell line or animal model using an enveloped virus whose lipid bilayer has been replaced with a lipid bilayer of a target animal.

### Background Art

In general, virion, which is a viral particle that has the ability to infect host cells, includes a core genome containing genetic material such as DNA or RNA, and a capsid, which is a protein shell that functions to protect such genetic material, and further includes an envelope in some cases. Types of viruses including an envelope include DNA viruses such as herpesvirus; RNA viruses such as flavivirus and retrovirus; and the like.

On the other hand, viruses (hepatitis viruses) that cause hepatitis may be classified into enveloped viruses (types B, C, and D) and non-enveloped viruses (types A and E). Hepatitis B virus (hereinafter referred to as 'HBV'), which is estimated to have an infection rate of about 350 million people worldwide, is a typical virus including an envelope and is a major cause of chronic liver disease. The HBV is a virus having a DNA genome that belongs to the hepadnavirus family and causes acute and chronic hepatitis. In particular, the prevalence of HBV, which was such that a percentage of chronically infected patients reached about 5% to 8% in Korea and China, has been decreased to some extent due to the development of vaccines. However, the truth is that a large number of chronic hepatitis patients still exist due to continuing outbreak of the mother-to-child vertical HBV infection. Chronic HBV infection causes liver cancer as well as hepatitis and cirrhosis. The WHO's research has demonstrated that about 80% of liver cancers is caused by chronic hepatitis B, and the incidence of liver cancer is about 300 times higher in HBV-infected people than non-HBV-infected people.

The HBV is classified into 8 genotypes whose genetic base sequences differ from one another by about 8% or higher, or 4 types of serotypes (adw, adr, ayw, and ayr, or the like) based on the two epitopes (d/y, w/r) in HBV surface antigen (HBsAg). Since the liver, which is a host of the hepatitis virus, induces immune tolerance, the virus can survive without rejection even in a case of histocompatibility complex-mismatched liver allografts.

On the other hand, for the hepatitis C virus (hereinafter referred to as 'HCV'), about 170 million people worldwide are infected with this virus. A high infection rate thereof is seen mainly in developed countries; and the HCV infection is highly associated with tattoos, administration of drugs such as narcotic drugs, and AIDS infection. Most HCV infections take a chronic course, and cause cirrhosis and liver cancer as complications thereof. HCV, which is an RNA genome virus, is a hepacivirus belonging to the Flaviviridae family and is divided into six genotypes (types 1 to 6), each genotype being divided into various subtypes. These subtypes of HCV are further differentiated in patients infected with HCV, and have quasispecies that exhibits genetic diversity.

In a case of chronic HBV infection, treatment is performed by oral administration of a nucleoside analog to inhibit viral proliferation, or by injection of interferon into an infected patient. The nucleoside analog has a better therapeutic effect than interferon, and has problems in that chronic use of the nucleoside analog causes HBV to develop resistance thereto and may also cause HBV to develop cross-resistance to other drugs. In addition, in a case of HCV infection, interferon monotherapy was attempted in the 1990s, and a treatment success rate thereof was only 10%. In the 2000s, combination therapy of peginterferon alpha and ribavirin resulted in an approximately 50% improvement in treatment success rate; however, this therapy caused severe drug adverse effects, and thus there was a difficulty in achieving practical treatment. Recently, direct antiviral agents (DAAs) have been developed, and oral administration of such drugs for 3 to 6 months made it possible to obtain a treatment success rate of about 80% to 90% with little adverse effects. However, since the DAA drugs are very expensive, there are problems that such drugs become a barrier to treatment for many patients and cause a huge loss of social costs. In addition, the HBV and HCV therapeutic agents developed so far are problematic in that such therapeutic agents do not ultimately kill the viruses but merely inhibit proliferation thereof.

The reason why no drugs capable of ultimately killing HBV and HCV have been developed as described above is that there is no method of actively culturing viruses with an *in vitro* cell culture system. In addition, there is no method available which enables production of an animal model by infection of small animals having a small size, such as mice, which can be traditionally used in infection experiments.

Due to such circumstances, for viruses, the life cycle of viral infection has not been accurately identified. In this regard, for HBV, the mechanism that can destroy cccDNA, which is a template for intranuclear viral genome replication, has not been elucidated; and for HCV, the mechanism for quasispecies that exhibits genetic diversity in infected patients has not been elucidated. For this reason, a perfect and ideal antiviral drug has not yet been developed.

The viruses, such as HBV and HCV, which include a lipid bilayer envelope, exhibit species specificity (or narrow host range) and organ tropism, and this made it difficult to achieve viral infection and culture *in vitro* and in small animals. Thus, so far, there has been no progress in development of therapeutic agents for the viruses. Therefore, *in vitro* and small animal models infected with the enveloped viruses including HBV and HCV are desperately needed as a tool to elucidate the life cycle and mechanism of pathogenesis of these viruses.

In the past few years, in the development of animal models for HBV and HCV, significant progress has been made such as construction of chimeric animal models capable of infection with the viruses. Nevertheless, lack of animal models presents a problem in that there is no clinically appropriate means for developing therapeutic agents suitable for viral hepatitis diseases. In particular, since HBV and HCV use only humans and chimpanzees as hosts, there is a problem that the viruses have such a limited host range, which presents a particular difficulty in producing *in vivo* models as well as *in vitro* models.

### Technical Problem

An object of the present invention is to provide a method for producing a virus-infected cell line model and a cell line produced by the same.

An object of the present invention is to provide a method for producing a virus-infected animal model and an animal model produced by the same.

Another object of the present invention is to provide a virus-infected cell line; and a method for screening a therapeutic candidate for a viral disease using a virus-infected animal model.

However, the technical problem to be solved by the present invention is not limited to the above-mentioned problems, and other problems which are not mentioned will be clearly understood by those skilled in the art from the following description.

### Solution to Problem

First of all, the present inventors have identified that in a case where a culture medium of a cell line is treated with a mixture of a bile acid or a bile acid derivative and an enveloped virus, the bile acid mechanically expands the lipid bilayer of the cell line, thereby causing destruction of the cell membrane, so that the viral infection occurs regardless of the virus' species specificity and tissue specificity. Furthermore, the present inventors have found that in a case where a cell line derived from a target animal is infected with an enveloped virus including a lipid bilayer in the same manner as above to produce an enveloped virus having the same lipid bilayer as that of the target animal cell line, and then the enveloped virus thus produced is injected into the target animal, it is possible to produce an animal model that can be very effectively used clinically; and thus have completed the present invention.

In an embodiment of the present invention, there is provided a method for producing a virus-infected cell line.

The method for producing a cell line, of the present invention, comprises steps of: mixing an enveloped virus including a lipid bilayer with a bile acid or a bile acid derivative; and adding, to a culture medium of a cell line, the virus mixed with the bile acid or the bile acid derivative.

The "enveloped virus including a lipid bilayer", as used herein, refers to an enveloped virus that includes a core genome containing genetic material such as DNA or RNA, and a capsid, which is a protein shell that functions to protect such genetic material; and an envelope containing a lipid bilayer.

In the present invention, the enveloped virus may include any virus as long as the virus further includes an envelope containing a lipid bilayer in addition to a capsid constituting the virus itself, and examples thereof include, but are not limited to, hepadnavirus, poxvirus, herpesvirus, and the like, which have DNA as genetic material; and coronavirus, filovirus, rhabdovirus, arenavirus, flavivirus, retrovirus, and the like, which have RNA as genetic material. Preferably, the virus may be a hepatitis virus, with hepatitis B virus (HBV) or hepatitis C virus (HCV) being more preferred. However, the virus is not limited thereto.

In the present invention, the cell line infected with the enveloped virus including a lipid bilayer means a cell line infected with the virus, for example, a cell line that is capable of maintaining its characteristics even in a case of being subcultured for a certain number or period of time *in vitro.* Preferably, the cell line may be immortalized so that it can be continuously subcultured *in vitro.* However, the present invention is not limited thereto.

The method for producing a cell line infected with an enveloped virus including a lipid bilayer, of the present invention, comprises the step of adding, to a culture medium of a cultured cell line, the virus mixed with the bile acid or bile acid derivative *in vitro.* Conventionally, it was difficult to induce infection of a cultured immortalized cell line with an enveloped virus including a lipid bilayer *in vitro;* however, in a case where a bile acid or a bile acid derivative is mixed with the virus and the mixture is used to treat a culture medium of the immortalized cell line, the lipid bilayer of the cell line is expanded so that the cell membrane is mechanically destroyed, and this makes it possible to very effectively induce infection of the immortalized cell line with the enveloped virus including a lipid bilayer even *in vitro.*

Due to such an effect, it is possible to produce the virus-infected cell line model using cell lines derived from various species and tissues, without limitation resulting from the viral species specificity and the viral specificity of having tropism to a specific tissue.

In the present invention, for humans, the bile acid is a primary bile acid synthesized from cholesterol in the liver, which is divided into cholic acid and chenodeoxycholic acid; and a second bile acid is produced from the primary bile acid through metabolism by intestinal bacteria in the large intestine, in which cholic acid is metabolized to deoxycholic acid or chenodeoxycholic acid to lithocholic acid. In addition, the bile acid may be conjugated with glycine or taurine in the liver, and thus converted into taurocholic acid and glycocholic acid (which are derivatives of cholic acid), taurochenodeoxycholic acid and glycochenodeoxycholic acid (which are derivatives of chenodeoxycholic acid), glycodeoxycholic acid and taurodeoxycholic acid (which are derivatives of deoxycholic acid), or glycolithocholic acid and taurolithocholic acid (which are derivatives of lithocholic acid). On the other hand, for rodents, muricholic acid is present as a secondary bile acid.

In the present invention, the bile acid or the bile acid derivative may be any one or more selected from the group consisting of cholic acid, chenodeoxycholic acid, deoxycholic acid, lithocholic acid, taurocholic acid, glycocholic acid, taurochenodeoxycholic acid, glycochenodeoxycholic acid, glycodeoxycholic acid, taurodeoxycholic acid, glycolithocholic acid, taurolithocholic acid, and muricholic acid, with taurochenodeoxycholic acid or taurocholic acid being preferred, in which, more preferably, taurochenodeoxycholic acid may be used for HBV, and taurocholic acid or taurochenodioxycholic acid may be used for HCV. However, the present invention is not limited thereto.

In the present invention, the cell line may be derived from normal or cancerous tissues and subcultured *in vitro,* and may be immortalized according to a conventional method. The cell line may be, for example, any one or more selected from the group consisting of a liver cell line, a lung cell line, a kidney cell line, a muscle cell line, a breast cell line, an immune T cell line, and a reproductive cell line, which may be preferably derived from humans or mice. However, the present invention is not limited thereto.

In the present invention, the human liver-derived cell line may be HepG2 cell line or Huh-7 cell line, and the mouse liver-derived cell line may be Hepalclc7 cell line or H4-II-E cell line. However, the present invention is not limited thereto.

In the present invention, the human-derived immortalized cell line may be, but is not limited to, any one or more selected from the group consisting of SNU-1327 cell line and A549 cell line, which are lung cancer cell lines, A-498 cell line, which is a kidney cancer cell line, MDA-MB-231 cell line, which is a breast cancer cell line, Jurkat cell line, which is an immune T cell line, and HeLa cell line, which is a cervical cancer cell line.

In the present invention, the mouse-derived immortalized cell line may be, but is not limited to, any one or more selected from the group consisting of LA-4 cell line, which is a lung cancer cell line, RAG cell line, which is a kidney cancer cell line, MTV/TM-011 cell line, which is a breast cancer cell line, EL4 cell line, which is an immune T cell line, and LC540 cell line, which is a testicular cancer cell line.

In another embodiment of the present invention, there is provided a virus-infected cell line.

The virus-infected cell line of the present invention may be produced by mixing an enveloped virus including a lipid bilayer with a bile acid or a bile acid derivative, and adding, to a culture medium of an immortalized cell line, the virus mixed with the bile acid or the bile acid derivative.

The virus-infected cell line of the present invention can be produced by the above-described production method, and thus description of the details related to the enveloped virus including a lipid bilayer, the bile acid derivative, the immortalized cell line, the production method, and the like is omitted to avoid excessive complexity of the specification due to repeated description.

In yet another embodiment of the present invention, there is provided a method for screening a therapeutic candidate for treatment of a viral disease.

The method for screening a therapeutic candidate for a viral disease comprises steps of: culturing the cell line infected with an enveloped virus including a lipid bilayer according to the present invention; and adding, to the cell line, a therapeutic candidate for a viral disease.

The virus-infected cell line used in the screening method of the present invention can be produced by the above-described production method, and thus description of the details related to the enveloped virus including a lipid bilayer, the bile acid, the bile acid derivative, the cell line, the production method, and the like is omitted to avoid excessive complexity of the specification due to repeated description.

In the present invention, the viral disease refers to a disease whose symptoms are caused by viral infection, in which the virus infects a tissue, to which it has tropism, so that a pathological change is induced in the tissue. The viral disease may include any disease as long as the disease causes a pathological change in a tissue, and may be preferably viral hepatitis. However, the viral disease is not limited thereto.

In the present invention, the viral hepatitis is hepatitis caused by viral infection, and there are hepatitis B, which accounts for 86% of patients with hepatitis, and hepatitis C, which accounts for 12% of the patients with hepatitis. The cause of the hepatitis B is divided into vertical transmission in which infection from mother to child occurs at birth, and acquired infection that occurs after birth. For the acquired infection, 95% of infected people have symptoms similar to cold and are naturally cured; however, 5% to 10% of the infected people are not cured and may develop into chronic hepatitis. Conversely, for the vertical transmission, most infected people develop into chronic hepatitis. On the other hand, the hepatitis C is self-limited in only about 10% to 15% of patients, which is relatively small as compared with hepatitis B, and 85% to 90% or higher of the patients develop into chronic hepatitis. In 10% to 20% of the patients with chronic hepatitis, a disease such as cirrhosis or liver cancer develops within 20 to 30 years.

In the present invention, for the viral disease, the infection is identified by checking relevant antigens and antibodies through serological testing. Therefore, to identify whether the therapeutic candidate for a viral disease has a therapeutic effect, the screening method of the present invention may further comprise a step of identifying presence or absence of a viral genome or surface antigen, in each of the cultured virus-infected cell line and the virus-infected cell line to which the therapeutic candidate for the viral disease has been added.

In the present invention, in a case where a level of the viral genome or surface antigen present is decreased in the virus-infected cell line model, to which the therapeutic candidate has been added, as compared with the virus-infected cell line, the candidate can be selected as a therapeutic substance for the viral disease.

In the present invention, in the step of identifying presence or absence of a viral genome or surface antigen, the identification may be made at a protein or nucleic acid level.

In the present invention, a method for detecting the protein or nucleic acid level is known. The detection may be performed through, for example, an antigen-antibody reaction, a substrate that specifically binds to a surface antigen, or a reaction with a nucleic acid, a peptide aptamer, or the like.

In the present invention, among the viral diseases, for the hepatitis B, presence or absence of antigens, such as HBsAg antigen, which is a surface antigen, HBeAg antigen, which is a secreted antigen, and HBcAg antigen, which is a core antigen, may be identified by known detection methods such as fluorescence-activated cell sorting, ELISA, Western blotting, timeresolved fluorescence immunoassay (TRFIA), and immunohistochemistry; and presence or absence of the HBV DNA may be examined through gene amplification experiments such as polymerase chain reaction and real-time polymerase chain reaction. However, the present invention is not limited thereto.

In the present invention, in a case of the hepatitis C, detection of HCV core protein and presence or absence of anti-HCV antibody may be identified through known detection methods, and presence or absence of HCV RNA may be examined through gene amplification experiments such as polymerase chain reaction and real-time polymerase chain reaction. However, the present invention is not limited thereto.

In still yet another embodiment of the present invention, there is provided a method for producing a virus-infected animal model.

The production method of the present invention comprises steps of: causing a lipid layer of an enveloped virus including a lipid bilayer to be replaced with a lipid layer derived from the target animal; and injecting, into the target animal, the virus for which the lipid layer replacement has been achieved.

The "enveloped virus including a lipid bilayer", as used herein, refers to a virus that includes a core genome containing genetic material such as DNA or RNA, and a capsid, which is a protein shell that functions to protect such genetic material; and an envelope containing a lipid bilayer.

In the present invention, the enveloped virus may include any virus as long as the virus further includes an envelope containing a lipid bilayer in addition to a capsid constituting the virus itself, and examples thereof include, but are not limited to, hepadnavirus, poxvirus, herpesvirus, and the like, which have DNA as genetic material; and coronavirus, filovirus, rhabdovirus, arenavirus, flavivirus, retrovirus, and the like, which have RNA as genetic material. Preferably, the virus may be a hepatitis virus, with hepatitis B virus (HBV) or hepatitis C virus (HCV) being more preferred. However, the virus is not limited thereto.

In the present invention, the step of causing a lipid layer of an enveloped virus including a lipid bilayer to be replaced with a lipid layer derived from the target animal may be performed through steps of: mixing the enveloped virus including a lipid bilayer with a bile acid or a bile acid derivative; and adding the virus mixed with the bile acid or the bile acid derivative to a culture medium of an immortalized cell line derived from the target animal. Preferably, the step may further comprise a step of isolating an enveloped virus including a target animal-derived lipid bilayer from the culture medium.

According to the above-described production method of the present invention, a human-derived lipid layer of a virus is replaced with a target animal-derived lipid layer, which can facilitate membrane fusion, thereby inducing a cross-species infection.

In the present invention, the step of isolating an enveloped virus may be performed by a conventional method such as, for example, centrifugation and chromatography. However, the present invention is not limited thereto.

In the present invention, the target animal may be a vertebrate animal other than humans, and may be preferably any one or more selected from the group consisting of mice, rats, rabbits, birds, cats, dogs, pigs, sheep, goats, deer, horses, and cattle, with mice or rats being most preferred. However, the target animal is not limited thereto.

In the present invention, in a case where a virus-infected animal model is produced using the vertebrate animal other than humans, the animal model may be used to understand various phenomena, such as infection path of the virus and disease development caused by the virus, and deduce significant results therefrom, because the animal model is similar to humans in terms of the immune system and the like.

In the present invention, the cell line may be derived from normal or cancerous tissues of the above-mentioned animals and subcultured *in vitro,* and may be immortalized according to a conventional method. The cell line may be, for example, any one or more selected from the group consisting of a liver cell line, a lung cell line, a kidney cell line, a muscle cell line, a breast cell line, an immune T cell line, and a reproductive cell line, and may be preferably Hepalclc7, which is a mouse liver cancer cell line, H4-II-E cell line, which is a rat liver cancer cell line, LA-4 cell line, which is a mouse lung cancer cell line, RAG cell line, which is a mouse kidney cancer cell line, MTV/TM-011 cell line, which is a mouse breast cancer cell line, EL4 cell line, which is a mouse immune T cell line, LC54 cell line, which is a mouse testicular cancer cell line, or the like.

In the present invention, for humans, the bile acid is a primary bile acid synthesized from cholesterol in the liver, which is divided into cholic acid and chenodeoxycholic acid; and a second bile acid is produced from the primary bile acid through metabolism by intestinal bacteria in the large intestine, in which cholic acid is metabolized to deoxycholic acid or chenodeoxycholic acid to lithocholic acid. In addition, the bile acid may be conjugated with glycine or taurine in the liver, and thus converted into taurocholic acid and glycocholic acid (which are derivatives of cholic acid), taurochenodeoxycholic acid and glycochenodeoxycholic acid (which are derivatives of chenodeoxycholic acid), glycodeoxycholic acid and taurodeoxycholic acid (which are derivatives of deoxycholic acid), or glycolithocholic acid and taurolithocholic acid (which are derivatives of lithocholic acid). On the other hand, for rodents, muricholic acid is present as a secondary bile acid.

In the present invention, the bile acid or the bile acid derivative may be any one or more selected from the group consisting of cholic acid, chenodeoxycholic acid, deoxycholic acid, lithocholic acid, taurocholic acid, glycocholic acid, taurochenodeoxycholic acid, glycochenodeoxycholic acid, glycodeoxycholic acid, taurodeoxycholic acid, glycolithocholic acid, taurolithocholic acid, and muricholic acid, with taurochenodeoxycholic acid or taurocholic acid being preferred, in which, more preferably, taurochenodeoxycholic acid may be used for HBV, and taurocholic acid or taurochenodioxycholic acid may be used for HCV. However, the present invention is not limited thereto.

In the present invention, in a case of being mixed with the virus, the bile acid or the bile acid derivative may be used at a concentration of 10 to 100 µmol/L, preferably 50 to 100 µmol/L, and more preferably 80 to 100 µmol/L. However, the present invention is not limited thereto. In a case where the bile acid is used at a concentration of lower than 10 µmol/L, the viral envelope may not be sufficiently removed; and in a case where the bile acid is used at a concentration of higher than 100 µmol/L, the viral genome stability and the like may be impaired.

In the present invention, in a case where the bile acid or the bile acid derivative is mixed with an enveloped virus including a lipid bilayer, and the mixture is added to a culture medium of a cell line derived from a target animal model, the lipid bilayer envelope of the virus is replaced with a lipid layer derived from the target animal model, so that the virus is capable of infecting an animal other than humans without species specificity.

In still yet another embodiment of the present invention, there is provided a virus-infected animal model.

In the present invention, the animal model may be produced by preparing a target animal other than humans, causing a lipid bilayer envelope of an enveloped virus including a lipid bilayer to be replaced with a lipid bilayer envelope derived from the target animal, and then injecting, into the target animal, the virus for which the lipid layer envelope replacement has been achieved.

In the present invention, the virus may be injected into a vein, such as a tail vein, of the target animal. However, the present invention is not limited thereto.

In the present invention, the animal model can be produced by the above-described production method, and thus description of the details related to the enveloped virus including a lipid bilayer, the bile acid, the bile acid derivative, the cell line, the target animal, the production method, and the like is omitted to avoid excessive complexity of the specification due to repeated description.

In still yet another embodiment of the present invention, there is provided a method for screening a therapeutic candidate for treatment of a viral disease.

The method for screening a therapeutic candidate for a viral disease, of the present invention, comprises steps of: rearing the virus-infected animal model according to the present invention; and adding, to the animal model, a therapeutic candidate for a viral disease.

The virus-infected animal model used in the screening method of the present invention can be produced by the above-described production method, and thus description of the details related to the enveloped virus including a lipid bilayer, the bile acid derivative, the immortalized cell line, the target animal, the production method, and the like is omitted to avoid excessive complexity of the specification due to repeated description.

In the present invention, the viral disease refers to an infection caused by viral infection, in which the virus infects a tissue, to which it has tropism, so that a pathological change is induced in the tissue. As described above, the viral disease may include any disease as long as the disease causes a pathological change in a tissue, and may be preferably viral hepatitis. However, the viral disease is not limited thereto.

In the present invention, the viral hepatitis is hepatitis caused by viral infection, and there are hepatitis B, which accounts for 86% of patients with hepatitis, and hepatitis C, which accounts for 12% of the patients with hepatitis. The cause of the hepatitis B is divided into vertical transmission in which infection from mother to child occurs at birth, and acquired infection that occurs after birth. For the acquired infection, 95% of infected people have symptoms similar to cold and are naturally cured; however, 5% to 10% of the infected people are not cured and may develop into chronic hepatitis. Conversely, for the vertical transmission, most infected people develop into chronic hepatitis. On the other hand, the hepatitis C is self-limited in only about 10% to 15% of patients, which is relatively small as compared with hepatitis B, and 85% to 90% or higher of the patients develop into chronic hepatitis. In 10% to 20% of the patients with chronic hepatitis, a disease such as cirrhosis or liver cancer develops within 20 to 30 years.

In the present invention, for the viral disease, the infection is identified by checking relevant antigens and antibodies through serological testing. Therefore, to identify whether the therapeutic candidate for a viral disease has a therapeutic effect, the screening method of the present invention may further comprise a step of identifying presence or absence of a viral genome or surface antigen, in each of the reared virus-infected animal model and the virus-infected animal model to which the therapeutic candidate for the viral disease has been added.

In a case where a level of the viral genome or surface antigen present is decreased in the virus-infected animal model to which the therapeutic candidate has been added, as compared with the reared virus-infected animal model of the present invention, the candidate can be selected as a therapeutic substance for the viral disease.

In the present invention, in the step of identifying presence or absence of a viral genome or surface antigen, the identification may be made at a protein or nucleic acid level.

In the present invention, a method for detecting the protein or nucleic acid level is known. The detection may be performed through, for example, an antigen-antibody reaction, a substrate that specifically binds to a surface antigen, or a reaction with a nucleic acid, a peptide aptamer, or the like.

In the present invention, among the viral diseases, for the hepatitis B, presence or absence of antigens, such as HBsAg antigen, which is a surface antigen, HBeAg antigen, which is a secreted antigen, and HBcAg antigen, which is a core antigen, may be identified by known detection methods such as fluorescence-activated cell sorting, ELISA, Western blotting, timeresolved fluorescence immunoassay (TRFIA), and immunohistochemistry; and presence or absence of the HBV DNA may be examined through gene amplification experiments such as polymerase chain reaction and a real-time polymerase chain reaction. However, the present invention is not limited thereto.

In the present invention, in a case of the hepatitis C, detection of HCV core protein and presence or absence of anti-HCV antibody may be identified through known detection methods, and presence or absence of HCV RNA may be examined through gene amplification experiments such as polymerase chain reaction and real-time polymerase chain reaction. However, the present invention is not limited thereto.

### Advantageous Effects of Invention

According to the present invention, for a human-derived lipid bilayer envelope of a virus, a bile acid or a bile acid derivative allows the human-derived lipid layer to be replaced with a target animal model-derived lipid layer; and this makes it possible to produce virus-infected cell line or animal model which can be effectively used clinically without limitation resulting from species specificity.

### Brief Description of Drawings

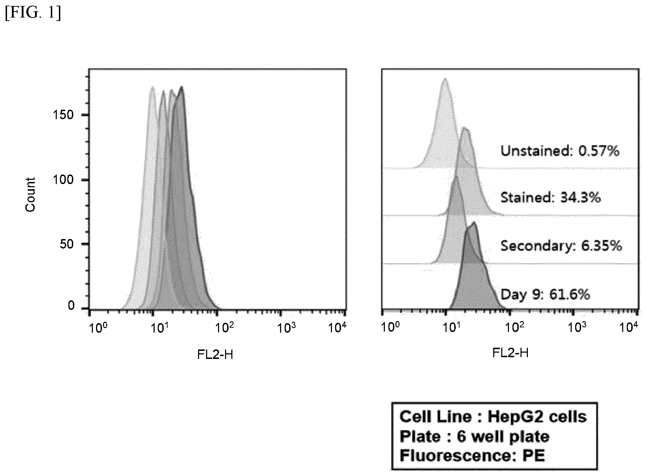

FIG. 1 illustrates results obtained by identifying, through fluorescence-activated cell sorting, HBcAg of HBV in HepG2 cell line, which is a human liver cancer cell line, according to an embodiment of the present invention.

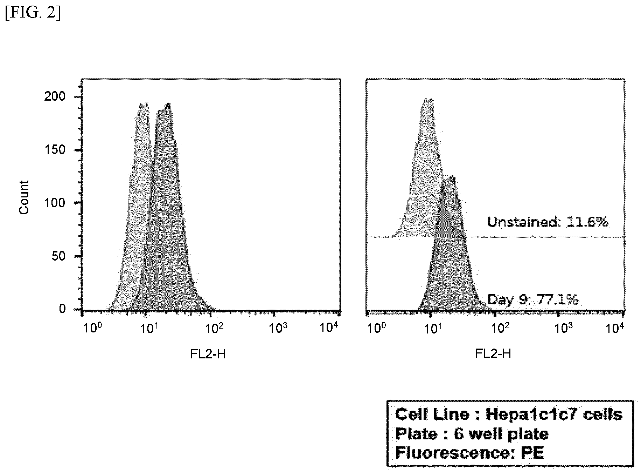

FIG. 2 illustrates results obtained by identifying, through fluorescence-activated cell sorting, HBcAg of HBV in Hepalclc7 cell line, which is a mouse liver cancer cell line, according to an embodiment of the present invention.

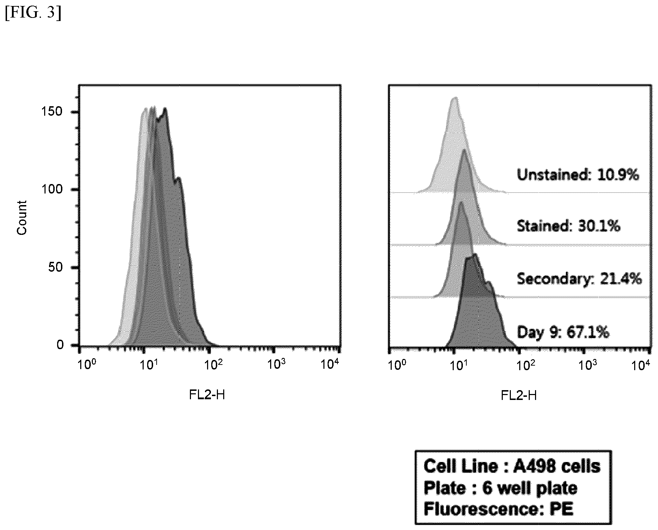

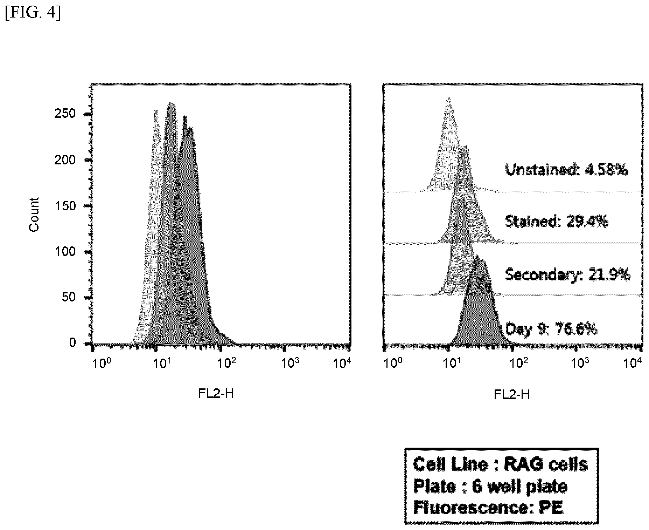

FIGS. 3 and 4 illustrate results obtained by identifying, through fluorescence-activated cell sorting, HBcAg of HBV in A498 cell line, which is a human kidney cancer cell line, or RAG cell line, which is a mouse kidney cancer cell line, according to an embodiment of the present invention.

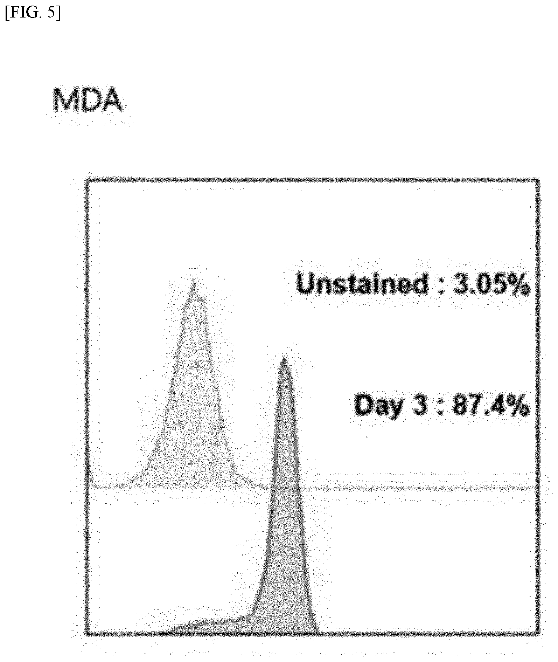

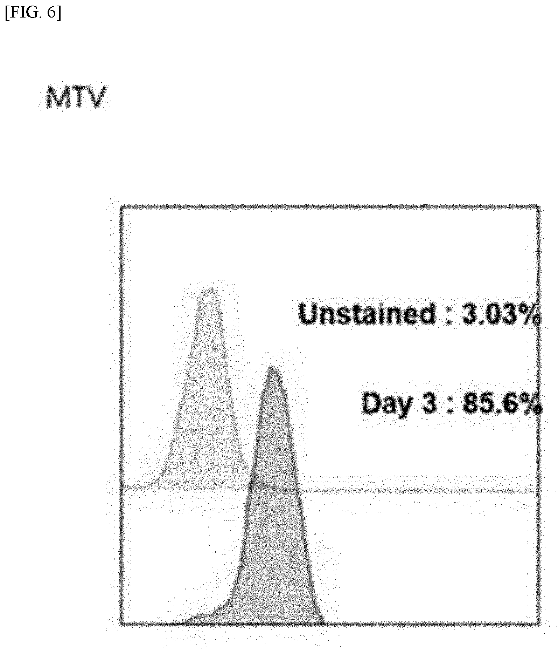

FIGS. 5 and 6 illustrate results obtained by identifying, through fluorescence-activated cell sorting, HBcAg of HBV in MDA-MB-231 cell line, which is a human breast cancer cell line, or MTV/TM-011 cell line, which is a mouse breast cancer cell line, according to an embodiment of the present invention.

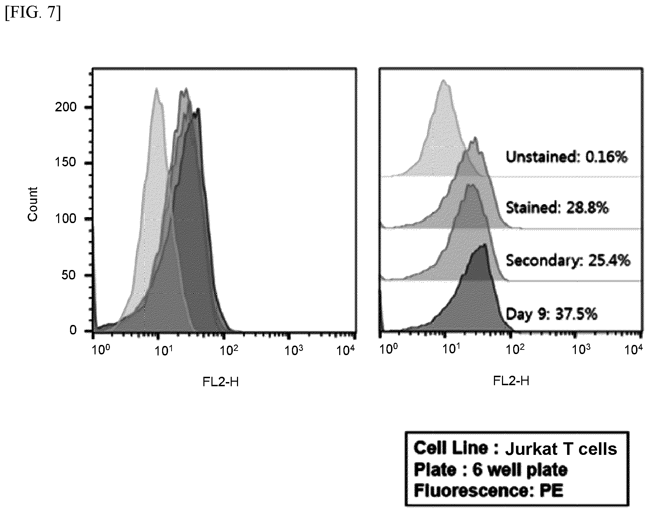

FIG. 7 illustrates results obtained by identifying, through fluorescence-activated cell sorting, HBcAg of HBV in Jurkat cell line, which is a human immune T cell line, according to an embodiment of the present invention.

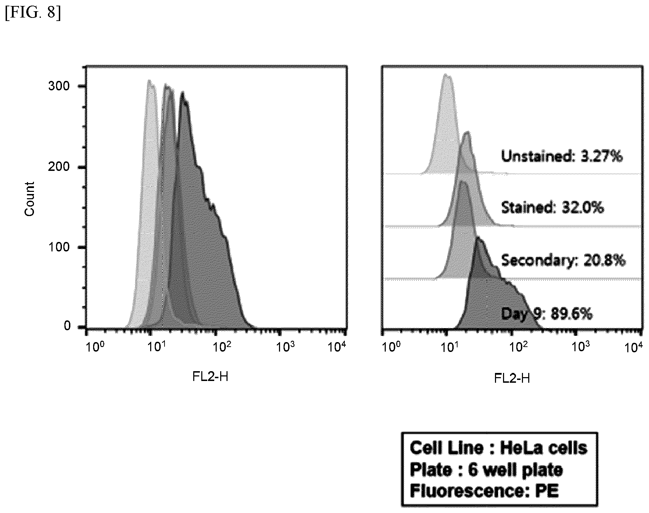

FIG. 8 illustrates results obtained by identifying, through fluorescence-activated cell sorting, HBcAg of HBV in HeLa cell line, which is a human uterine cell line, according to an embodiment of the present invention.
FIGS. 9 to 16 illustrate immunostaining results, according to an embodiment of the present invention.
FIGS. 17 to 22 illustrate results obtained by performing H&E staining of organs of a virus-infected animal model, according to an embodiment of the present invention.

### Detailed Description of Invention

In an embodiment of the present invention, there is provided a method for producing a virus-infected cell line, comprising steps of: mixing an enveloped virus including a lipid bilayer with a bile acid or a bile acid derivative; and adding, to a culture medium of a cell line, the virus mixed with the bile acid or the bile acid derivative.

In another embodiment of the present invention, there is provided a method for producing a virus-infected animal model, comprising steps of: preparing a target animal other than humans; causing a lipid layer of an enveloped virus including a lipid bilayer to be replaced with a lipid layer derived from the target animal; and injecting, into the target animal, the virus for which the lipid layer replacement has been achieved.

Hereinafter, the present invention will be described in more detail by way of examples. These examples are only for describing the present invention in more detail, and it will be apparent to those skilled in the art that according to the gist of the present invention, the scope of the present invention is not limited by these examples.

### Examples

### [Preparation Example 1] Isolation of hepatitis virus

Normal serum (hepatitis B virus (HBV)- and hepatitis C virus (HCV)-negative) and hepatitis virus-infected serum (HBV- or HCV- positive) were received from Wonju Severance Christian Hospital (Ethics Committee Approval No. CR316312).

Each of the above-mentioned sera was added to a tube having 3 ml of a cushion buffer that contains 20 g/L of sucrose, 50 mmol/L of Tris-HCl (pH 7.5), and 30 mmol/L of NaCl. The mixture was centrifuged for 1 hour at 220,000 g. Then, the supernatant was removed from the tube, and 5 ml of phosphate-buffered saline (PBS) solution was added to the tube for dilution of HBV or HCV.Then, the HCV RNA or the HBV DNA contained in the dilution was measured through real-time PCR using the Roche's COBAS TaqMan system to determine a concentration of the isolated virus.

### [Preparation Example 2] Cell line culture

The cell lines used in the examples were purchased from the Korea Cell Line Bank, and cell culture was performed using the culture medium and culture method specified by the Korea Cell Line Bank.

For rodent cell lines, the following cell lines were used: mouse C57L (The Jackson Laboratory, USA)-derived Hepalclc7 cell line or H4-II-E cell line, which is a liver cancer cell line; RAG which is a kidney cancer cell line; EL4 which is an immune T cell line; LC540 which is a testicular cancer cell line; and LA-4 which is a lung cancer cell line.

For human cell lines, the following cell lines were used: HepG2 or Huh-7 which is a liver cancer cell line; A-498 which is a kidney cancer cell line; MDA-MB-231 which is is a breast cancer cell line; A549 which is a lung cancer cell line; Jurkat which is an immune T cell line; and HeLa which is a uterine cancer cell line. As a control, PLC/PRF5 cell line, which is a liver cell line, was used.

Here, after thawing, the cells were subcultured three times for cell stabilization, and then cultured for 24 hours. Thereafter, hepatitis viral infection was performed.

### [Example 1] Production of virus-infected cell line model

For production of enveloped virus-infected cell line models, HBV (1 × 10⁶ copies/ml/well of HBV) isolated in Preparation Example 1 was mixed with 100 µmol/L of taurochenodeoxycholic acid (hereinafter referred to as 'tCDCA'), and HCV (1 × 10⁶ IU/ml/well of HCV) isolated in Preparation Example 1 was mixed with tCDCA or taurocholic acid (hereinafter referred to as 'tCA'). Here, tCA was mixed with HCV and used to infect mouse hepatocytes; and tCDCA was used in all other cases.

A cell line (HepG2, Huh-7, A549, A-498, MDA-MB-231, Jurkat, HeLa, Hepa1c1c7, EL4, H4-II-E, LA-4, LC540, or RAG cell line) at 5 × 10⁶ cells/ml was dispensed into each well of a 6-well plate, and culture was performed for 24 hours. Then, the tCDCA or tCA solution mixed with HBV or HCV was added thereto, and culture was further performed for 24 hours. Here, as a control, a group, to which only HBV or HCV was added without the tCDCA or tCA solution, was used. Then, washing with phosphate buffered saline (PBS) solution was performed three times, and the medium was replaced with cell culture medium (DMEM) that does not contain the virus. Finally, the cell culture medium was treated with 100 µmol/L of tCDCA or tCA, and culture was further performed for 3 days, thereby producing a virus-infected cell line model (hereinafter referred to as 'infected cell line').

### [Example 2] Verification of infected cell line by HBV

Levels of HBsAg and HBV DNA present were measured in each of the HBV-infected cell lines produced in Example 1 and the culture medium collected therefrom. Here, the measurement of HBsAg was performed using an Elecsys HBsAg II CLIA kit (REF: 07251076119, Roche, Switzerland) by an experimental method provided by the manufacturer, and the measurement of HBV DNA was performed by requesting a specialized inspection agency (Biogeno Korea Co., Ltd., Korea) that utilizes the Roche's COBAS TaqMan system.

In addition, FACS analysis was performed using an anti-HBcAg antibody, to measure a level of HBcAg present. Specifically, 100 µl/well of anti-HBcAg antibody (10E11) (cat no. MA1-7608; Thermo Fisher, USA) was added to a micro ELISA plate, and the reaction was allowed to proceed at 4°C overnight. Then, 200 µl/well of 1X ELISA/ELISPOT Diluent (Invitrogen, USA) was additionally added thereto, and the reaction was allowed to proceed for 1 hour. Each of the HBV-infected cell lines produced in Example 1 was dispensed into each well, in which the reaction had been completed, and the reaction was allowed to proceed for 2 hours. Then, the anti-HBcAg antibody (10E11) diluted in a ratio of 1:30 with 100 µl of 1X ELISA/ELISPOT Diluent was added to the plate, and the reaction was allowed to proceed for 1 hour. Then, the additional reaction with 100 µl of HRP-conjugated anti-rabbit IgG was allowed to proceed for 30 minutes. For detection of HBcAg, FACSCalibur was used, and the results are shown in Tables 1 and 2. Here, as a positive control, PLC/PRF5 cell line (human liver-derived cell line that releases HBV surface antigen) was used, and levels of HBsAg and HBcAg present therein were measured.

**[Table 1]**

| | Negative Control* | PLC/PRF5** | HepG2 | Huh-7 | A498 | MDA-MB-231 | Jurkat | HeLa |
|---|---|---|---|---|---|---|---|---|
| HBcAg (Index) | Not detected | 32.7% | 61.6% | 69.8% | 67.1% | 87.4% | 37.5% | 89.6% |
| HBsAg (Index) | 1.00 or lower | 71.49 ± 1.53 | 474.70 ± 176.30 | 26.02 ±5.97 | 5.12 ±0.19 | 169 | 1081 ±329 | 61.35 |
| HBV DNA (copies/ml) | 58.2 or lower | 65,100±9,098 | 3,590,000 ±1,010,000 | 89,000 ±1,836 | 73,500 ±23,292 | 116 | 868,000 ±88,963 | 359 |
| * Negative control: HepG2 cell line group, treated with only HBV | | | | | | | | |
| ** Positive control: Group, not treated with virus and tCDCA | | | | | | | | |

**[Table 2]**

| | Control*** | Hepalclc7 | H4-II-E | RAG | EL4 | LC540 |
|---|---|---|---|---|---|---|
| HBcAg (Index) | Not detected | 77.1% | 32.6% | 76.6% | 66.9% | - |
| HBsAg (Index) | 1.00 or lower | 508.26 ±107.6 | 22.73 ±4.39 | 21 (744) | 192.7 ±30.60 | 3.06 ±0.44 |
| HBV DNA (copies/ml) | 58.2 or lower | 7,036,667 | 73,000±51,929 | 11,700 (622, 000) | 673,000 ±60,053 | 149,000 ±29,866 |
| *** Control: Hepalclc7 cell line group, treated with only HBV | | | | | | |

As shown in Tables 1 and 2, it was identified that HBsAg and HBcAg were detected in the culture medium of HepG2, which is a human liver cancer cell line, and Hepalclc7, which is a mouse liver cancer cell line, and at least 100 copies of HBV DNA were present therein. Likewise, as a result of checking HBcAg of HBV through FACS, it was identified that the cell lines were infected with the tCDCA-treated HBV as shown at the genome level.

In addition, it was identified that HBsAg and HBcAg were detected in the culture medium of each of cell lines (A498, MDM-MB-231, Jurkat, HeLa, H4-II-E, RAG, EL4, and LC540) derived from various organs other than human and mouse liver, and HBV DNA was present therein.

From the above results, it can be seen that in a case where a cell culture medium of each of human and mouse liver cancer cell lines was treated with a bile acid or a bile acid derivative and HBV together, HBV can be induced to infect the cell lines, indicating that the bile acid or the bile acid derivative can eliminate HBV's species specificity (or narrow host range) and organ tropism so that HBV is induced to infect cell lines derived from various species and tissues other than the liver.

### [Example 3] Verification of infected cell line by HCV

In Example 1, the levels of HCV core and HCV RNA present were measured in each of the HCV-infected cell lines produced in Example 1 and the culture medium collected therefrom. Specifically, measurement of HCV RNA was performed by requesting a specialized inspection agency (Biogeno Korea Co., Ltd., Korea) that utilizes the Roche's COBAS TaqMan system. The results are shown in Tables 3 and 4. Here, as a control, a group treated with only HCV without a bile acid derivative was used.

**[Table 3]**

| | Control | HepG2 | Huh-7 | A549 | A498 | MDA-MB-231 | Jurkat |
|---|---|---|---|---|---|---|---|
| HCV RNA (IU/ml) | 15 or lower | 85.233 | 628.066 | 4550 | 610 | 605 | 3326.667 |

**[Table 4]**

| | Control | Hepalclc7 | H4-II-E | EL4 | LC540 |
|---|---|---|---|---|---|
| HCV RNA (IU/ml) | 15 or lower | 3430 | 197.33 | 1669 | 21.7 |

As shown in Tables 3 and 4, it was identified that the HCV RNA was present at 1000 IU/ml or higher in the culture medium of each of HepG2 cell line, which is a human liver cancer cell line, and Hepalclc7 cell line, which is a mouse liver cancer cell line. In addition, it was identified that the HCV RNA was also detected in human- and mouse-derived cell lines that are derived from organs other than the liver.

From the above results, it can be seen that, similar to the results for HBV, in a case where a cell culture medium of each of human and mouse liver cancer cell lines was treated with a bile acid or a bile acid derivative and HCV together, HCV can be induced to infect the cell lines. Furthermore, it can be seen that the bile acid or the bile acid derivative can eliminate HCV's species specificity or narrow host range and organ tropism so that HCV is induced to infect cell lines derived from various species and tissues.

From the results described in Examples 2 and 3, it can be seen that in a case where a cell line is treated with a bile acid or a bile acid derivative and HBV or HCV together, the bile acid or the bile acid derivative expands the lipid bilayer of the cell line, thereby causing mechanical destruction of the cell membrane, so that the hepatitis viral infection can be very effectively induced.

### [Example 4] Production of virus derived from mouse liver cancer cell line

For production of an enveloped virus-infected animal model, HBV (1 × 10⁸ copies/ml/well of HBV) or HCV (1 × 10⁸ IU/ml/well of HCV) isolated in Preparation Example 1 was mixed with 100 µmol/L of tCDCA.

Hepalclc7 cell line at 1 × 10⁵ cells/ml was dispensed into a 6-well plate, and culture was performed for 24 hours. Then, the tCDCA solution mixed with HBV or HCV was added thereto, and culture was further performed for 24 hours. Here, as a control, a group to which only the tCDCA solution was added was used. Then, washing with PBS solution was performed three times, and the medium was replaced with a cell culture medium that does not contain the virus. Subsequently, culture was further performed for 3 days. Then, viruses were isolated from the culture medium obtained from the Hepalclc7 cell line in the same manner as in Preparation Example 1.

Here, in the isolated viruses, the lipid bilayer envelope was replaced with one derived from the Hepalclc7 cell line. Thus, the viruses were designated mouse liver cancer cell line-derived HBV (hereinafter referred to as 'cHBVcc') and mouse liver cancer cell line-derived HCV (hereinafter referred to as 'cHCVcc'), respectively. Then, the concentration of the respective viruses was checked through real-time PCR using the COBAS TaqMan system (Roche, Switzerland).

### [Example 5] Viral stability of cHBVcc and cHCVcc

Viral stability of cHBVcc and cHCVcc obtained in Example 4 was checked in various environments, before identifying their cross-infectivity.

Human serum (HS), mouse serum (MS), and a culture medium of a cell line were treated with the cHBVcc or cHCVcc, and then levels of HBsAg and HBV DNA present in the sera or the culture medium were measured in the same manner as in Example 2. The results are shown in Table 5. Here, as a control, the serum of a patient with chronic hepatitis was used.

**[Table 5]**

| | Control | Human serum | Mouse serum | Cell culture medium |
|---|---|---|---|---|
| HBsAg (Index) | 2,897 | Negative(negative) | Negative | Negative |
| HBV DNA (copies/ml) | 4.36 × 10⁸ | 224,000 | 31,400 | 12,300 |

As shown in Table 5, it can be seen that as compared with the control, the cHBVcc, for which replacement with a mouse liver cancer cell line-derived lipid bilayer had been achieved, exhibited negative surface antigenicity and also had a greatly decreased amount of genome; however, the amount of genome corresponds to a value well above the minimum infectious dose, and thus even the cHBVcc shows stability in terms of viral envelope state, so that the cHBVcc can very effectively infect a target animal such as humans or mice.

### [Example 6] Identification of viral cross-infection in allogeneic cells

### [6-1] Identification of cross-infectivity of cHBVcc (1)

Cross-infectivity of the cHBVcc obtained in Example 4 was identified in Hepalclc7 cell line. Specifically, the Hepalclc7 cell line was treated with a mixed solution (cHBVcc + tCDCA), obtained by mixing the cHBVcc in Example 4 with 100 µmol/L of tCDCA, or cHBVcc, and then HBsAg and HBV DNA were measured in the same manner as in Example 2. The results are shown in Table 6. Here, as a control, the HBV (wild-type HBV) obtained in Preparation Example 1 was used.

**[Table 6]**

| | Wild-type HBV | HBV + tCDCA | cHBVcc | cHBVcc + tCDCA |
|---|---|---|---|---|
| HBsAg (Index) | Negative | 18.61 | 7.61 | Negative |
| HBVDNA (copies/ml) | Negative | 657 | 190,000 | 130,000 |

As shown in Table 6, it was identified that in the Hepalclc7 cell line, no infection occurred in a case of being treated with the wild-type HBV, and infection occurred in a case of being treated with a mixture with tCDCA.

Furthermore, it was identified that in the Hepalclc7 cell line, infection with cHBVcc occurred in a case of being treated with the cHBVcc whose lipid bilayer had been replaced with one derived from the Hepa1c1c7 cell line even in the absence of tCDCA (cHBVcc).

From the above results, it can be seen that even in an environment where a bile acid or a bile acid derivative is not present, the virus, for which replacement of lipid bilayer envelope has been achieved using a bile acid or a bile acid derivative, can infect other allogeneic cells, and furthermore, can very effectively infect an animal corresponding to the same species as the cells.

### [6-2] Identification of cross-infectivity of cHCVcc (2)

Cross-infectivity of the cHCVcc obtained in Example 4 was identified in Hepalclc7 cell line. Specifically, the Hepalclc7 cell line was treated with a mixed solution (cHCVcc + tCDCA), obtained by mixing the cHCVcc in Example 4 with 100 µmol/L of tCDCA, or cHCVcc, and then HCV RNA was measured in the same manner as in Example 3. The results are shown in Table 7. Here, as a control, the HCV (wild type HCV) obtained in Preparation Example 1 was used.

**[Table 7]**

| | Wild-type HCV | HCV + tCDCA | cHCVcc | cHCVcc + tCDCA |
|---|---|---|---|---|
| HCV RNA (IU/ml) | Negative | 3,403 | 1330 | 1330 |

As shown in Table 7, it was identified that in the Hepalclc7 cell line, no infection with the wild-type HCV occurred in a case of being injected with only the wild-type HCV, and infection occurred in a case of being treated with a mixture with tCDCA.

Furthermore, it was identified that in the Hepalclc7 cell line, infection occurred in a case of being treated with the cHCVcc whose lipid bilayer had been replaced with one derived from the Hepa1c1c7 cell line even in the absence of tCDCA.

From the above results, it can be seen that even in an environment where a bile acid or a bile acid derivative is not present, the virus, for which replacement of lipid bilayer envelope has been achieved using a bile acid or a bile acid derivative, can infect xenogeneic cells, and furthermore, can very effectively infect an animal whose species is the same as that from which the cells are derived.

### [6-3] Identification of cross-infectivity (3)

To HepG2 cell line was added a solution obtained by mixing HBV (1 × 10⁸ copies/ml/well of HBV) or HCV (1 × 10⁸ IU/ml/well of HCV) isolated in Preparation Example 1 with 100 µmol/L of tCDCA. Then, the same method as in Example 4 was performed to obtain HBV and HCV for which replacement of lipid bilayer envelope had been achieved. Then, HepG2 cell line was treated with 100 µmol/L of bile acid-mixed solution (HBV + tCDCA or HCV + tCDCA), and then levels of HBsAg and HBV DNA or HCV RNA were measured in the same manner as in Examples 2 and 3. The results are shown in Tables 8 and 9.

**[Table 8]**

| | HBV | HBV+ tCDCA |
|---|---|---|
| HBsAg (Index) | 5.99±0.89 | 4.67±0.58 |
| HBVDNA (copies/ml) | 48000±18504 | 76400±19552 |

**[Table 9]**

| | HCV | HCV + tCDCA |
|---|---|---|
| HCV RNA (IU/ml) | 15 or lower | 182 |

As shown in Table 8, in the presence of tCDCA, the HepG2 cell line was infected with the HBV whose lipid bilayer envelope is derived from the HepG2 cell line, and thus HBsAg and HBV DNA were detected therein. Furthermore, as shown in Table 9, it was identified that the HepG2 cell line was infected with the HCV whose lipid bilayer envelope is derived from the HepG2 cell line, and thus 182 IU/ml of RNA was present therein.

From the above results, it can be seen that the virus, for which replacement of lipid bilayer envelope has been achieved using a bile acid or a bile acid derivative, can very efficiently infect allogeneic cells.

### [6-4] Identification of cross-infectivity (4)

To HepG2 cell line was added a solution obtained by mixing HBV (1 × 10⁸ copies/ml/well of HBV) or HCV (1 × 10⁸ IU/ml/well of HCV) isolated in Preparation Example 1 with 100 µmol/L of tCDCA. Then, the same method as in Example 4 was performed to obtain HBV and HCV for which replacement of lipid bilayer envelope had been achieved. Then, Hepalclc7 cell line was treated with 100 µmol/L of bile acid-mixed solution (HBV + tCDCA or HCV + tCDCA), and then levels of HBsAg and HBV DNA or HCV RNA were measured in the same manner as in Examples 2 and 3. The results are shown in Tables 10 and 11.

**[Table 10]**

| | HBV | HBV+ tCDCA |
|---|---|---|
| HBsAg (Index) | 3.28±0.89 | 19.32±1.85 |
| HBVDNA (copies/ml) | 47000±13200 | 292000±38553 |

**[Table 11]**

| | HCV | HCV + tCDCA |
|---|---|---|
| HCV RNA (IU/ml) | 15 or lower | 182.3 |

As shown in Tables 10 and 11, in the presence of tCDCA, the Hepa1c1c7 cell line was infected with the HBV whose lipid bilayer envelope is derived from the HepG2 cell line, and thus HBsAg and HBV DNA were detected therein. Furthermore, as shown in Table 11, it was identified that the Hepalclc7 cell line was infected with the HCV whose lipid bilayer envelope is derived from the HepG2 cell line, and thus 182.3 IU/ml of RNA was present therein.

From the above results, it can be seen that the virus, for which replacement of lipid bilayer envelope has been achieved using a bile acid or a bile acid derivative, can infect xenogeneic cells, and further can infect an animal whose species is different from that from which the cells are derived.

### [6-5] Identification of cross-infectivity (5)

To Hepalclc7 cell line was added a solution obtained by mixing HBV (1 × 10⁸ copies/ml/well of HBV) or HCV (1 × 10⁸ IU/ml/well of HCV) isolated in Preparation Example 1 with 100 µmol/L of tCDCA. Then, the same method as in Example 4 was performed to obtain HBV and HCV for which replacement of lipid bilayer envelope has been achieved. Then, HepG2 cell line was treated with 100 µmol/L of bile acid-mixed solution (HBV + tCDCA or HCV + tCDCA), and then levels of HBsAg and HBV DNA or HCV RNA were measured in the same manner as in Examples 2 and 3. The results are shown in Tables 12 and 13.

**[Table 12]**

| | HBV | HBV+ tCDCA |
|---|---|---|
| HBsAg (Index) | Not detected | 32.5±1.85 |
| HBVDNA (copies/ml) | 15.8 or lower | 392000±32203 |

**[Table 13]**

| | HCV | HCV + tCDCA |
|---|---|---|
| HCV RNA (IU/ml) | 15 or lower | 300 |

As shown in Table 12, in the presence of tCDCA, the HepG2 cell line was infected with the HBV whose lipid bilayer envelope is derived from the Hepalclc7 cell line, and thus HBsAg and HBV DNA were detected therein. Furthermore, as shown in Table 13, it was identified that the Hepalclc7 cell line was infected with the HCV whose lipid bilayer envelope is derived from the HepG2 cell line, and thus 300 IU/ml of RNA was present therein.

From the above results, it can be seen that the virus, for which replacement of lipid bilayer envelope has been achieved using a bile acid or a bile acid derivative, can very efficiently infect allogeneic cells.

### [Example 7] Production and verification of HBV and HCV virus-infected animal models

The cHBVcc or cHCVcc prepared in Example 4 was injected in an amount of 100 µl (1 × 10⁸ genome equivalents) into the tail vein of mouse C57L, and the mouse was reared for 1 week to produce a virus-infected animal model (hereinafter referred to as 'infected animal'). Here, as a control, a mouse, into which human-derived wild-type HBV was injected in an amount of 100 µl, was used.

### [7-1] Virus test in serum

The serum was obtained from each infected animal, and levels of HBsAg and HBV DNA present in the serum were measured. Here, as a control, the serum from a mouse, into which wild-type HBV having a human-derived lipid bilayer envelope was injected, was used. Measurement of HBsAg and HBV DNA or HCV RNA was performed in the same manner as in Examples 2 and 3, and the results are shown in Tables 14 and 15.

**[Table 14]**

| | Control | mHBVcc |
|---|---|---|
| HBsAg | 0.1 (negative) | 1.2 |
| HBVDNA (copies/ml) | 58.2 or lower (negative) | 585 |

**[Table 15]**

| | Control | mHCVcc |
|---|---|---|
| HCV RNA (IU/ml) | 15 or lower | 180 |

As shown in Table 14, it was identified that the level of HBsAg in the infected animal's serum was 1.2 Index, and HBV DNA was present in 585 copies/ml. In addition, as shown in Table 15, it was identified that for HCV RNA, the control exhibited 15 IU/ml or lower, whereas the mHCVcc exhibited 180 IU/ml.

From the above results, it can be seen that injection of cHBVcc and cHCVcc can induce HBV infection in a small animal, such as mice, which is species other than humans.

### [7-2] Liver function test

In the infected animal, to check whether hepatitis caused by viral infection had occurred, a liver function test, in which a level of alanine aminotransaminase (ALT) in serum is measured, was performed by a conventional method in the art.

As a result, the ALT in the infected animal injected with cHBVcc was measured to be 150 U/L.

From the above results, it can be seen that since the normal ALT value has 34 or lower, injection of cHBVcc can cause HBV infection in the mouse that is an infected animal, indicating that hepatitis has occurred in the infected animal.

### [7-3] Tissue biopsy

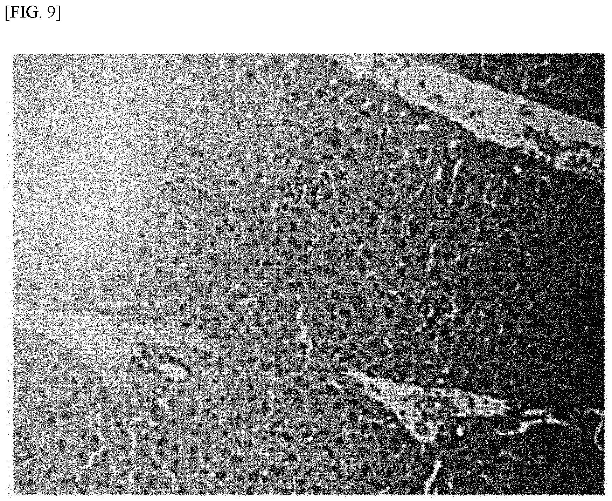

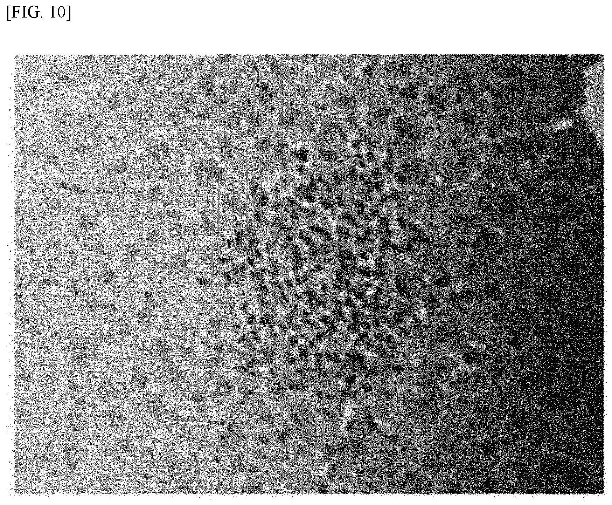

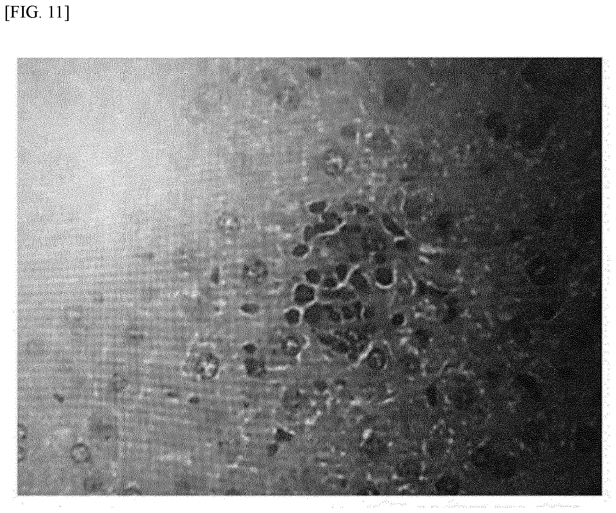

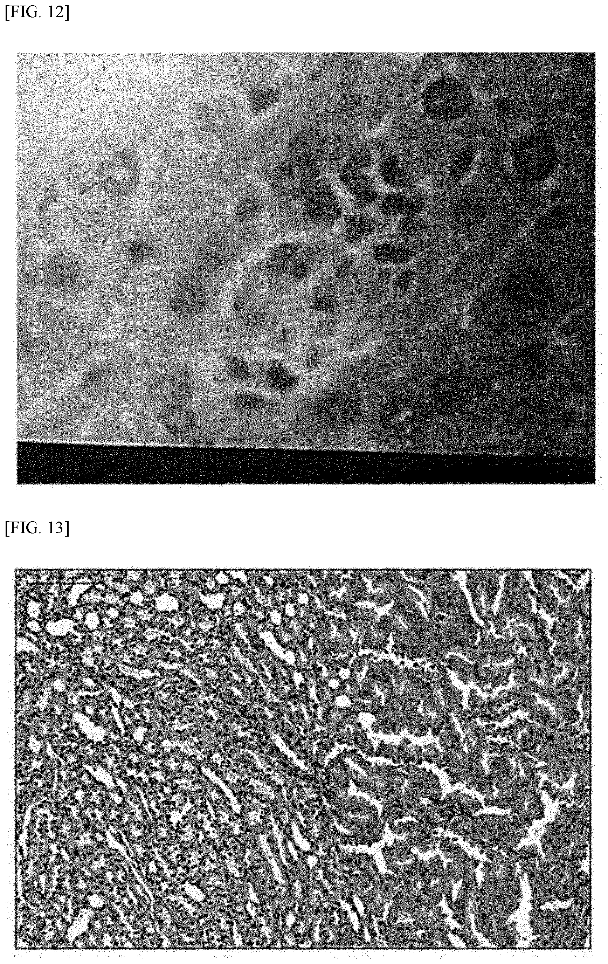

Liver tissues were excised from the infected animal, fixed using 10% formalin, and then embedded in paraffin. The paraffin-embedded tissue was cut into 4 µm-thick sections, and rehydration and paraffin removal were performed. Then, H&E staining was performed for the sections through a conventional method, and the results are illustrated in FIGS. 9 to 16.

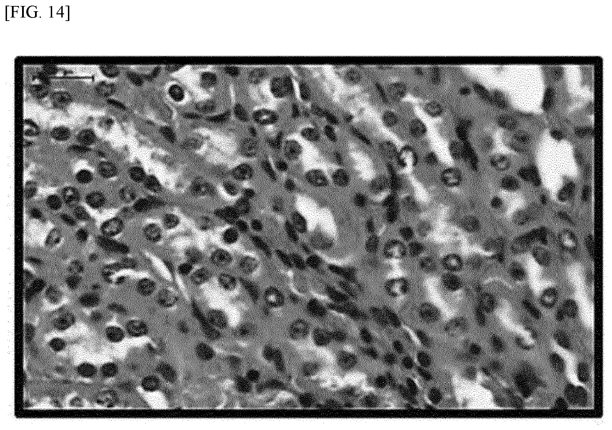

As shown in FIGS. 9 and 14, it was identified that inflammation occurred in view of the fact that increased infiltration of monocytes (lymphocytes) was observed at several places in the hepatic parenchyma, and infiltration of monocytes and balloon-shaped mass were also observed in the peri-portal area, which was similar to histological findings of human acute hepatitis B.

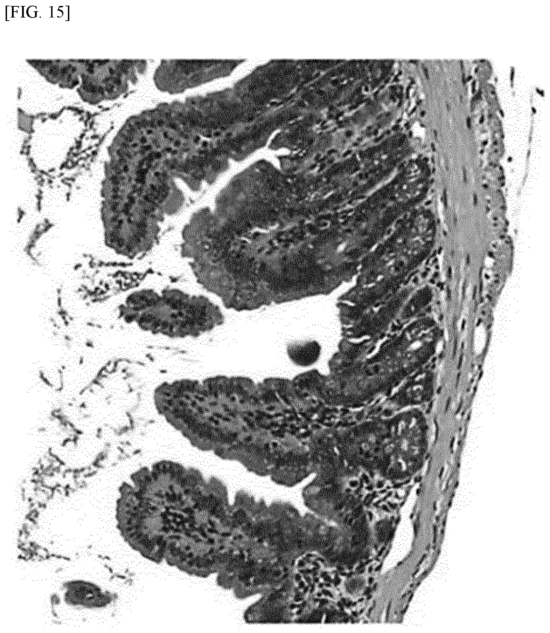

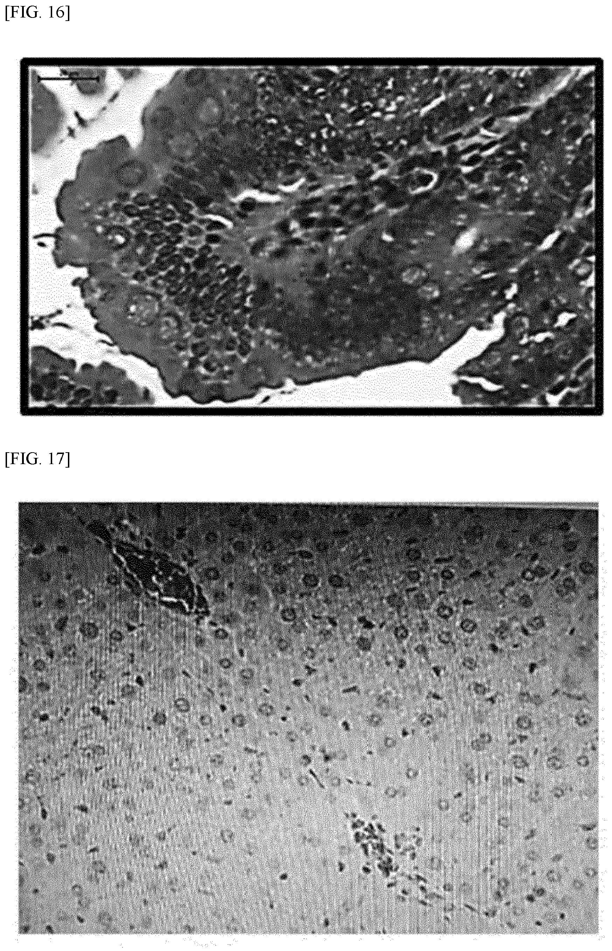

As illustrated in FIGS. 15 and 16, it was identified that monocytes (lymphocytes) were infiltrated into the villi of the ileum.

From the above results, it can be seen that the cHBVcc according to the present invention can infect a rodent and such an infection can lead to acute inflammation caused by HBV infection in renal and iliac tissues as well as liver tissues.

### [7-4] Immunostaining test

Liver tissues were excised from the infected animal, fixed using 10% formalin, and then embedded in paraffin. The paraffin-embedded tissue was cut into 4 µm-thick sections, and rehydration and paraffin removal were performed. Then, the tissue was reacted with an antibody against HBcAg, which is an HBV core antigen, and reacted with a secondary antibody to which a substance (biotin) that can be visualized is bound. Then, staining with Hoechst 33258 was performed for 15 minutes. Then, mounting was performed through 50% glycerol, and observation was performed under a microscope. The results are illustrated in FIGS. 9 to 14.

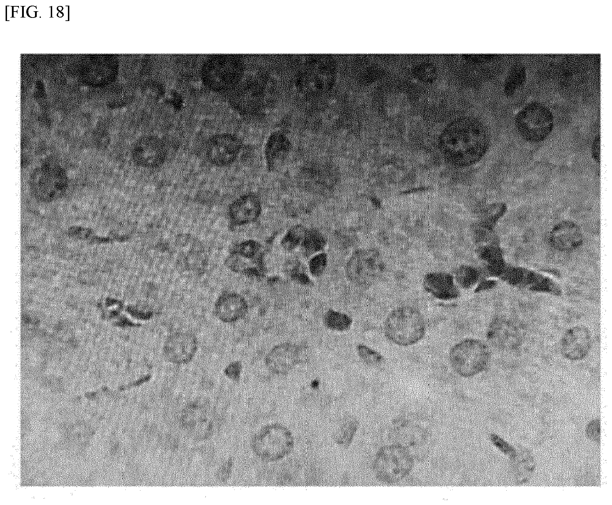

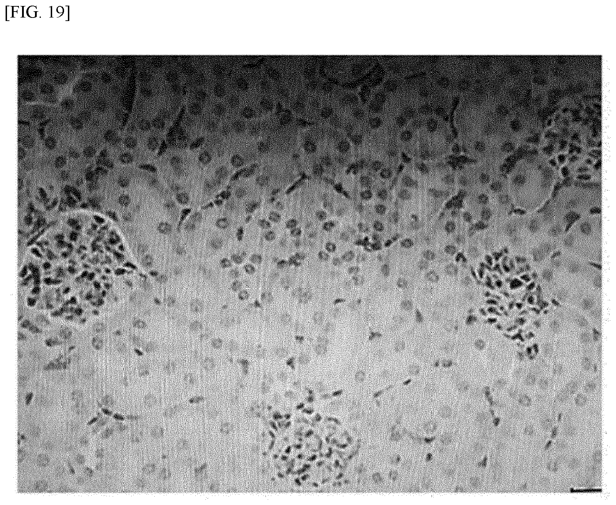

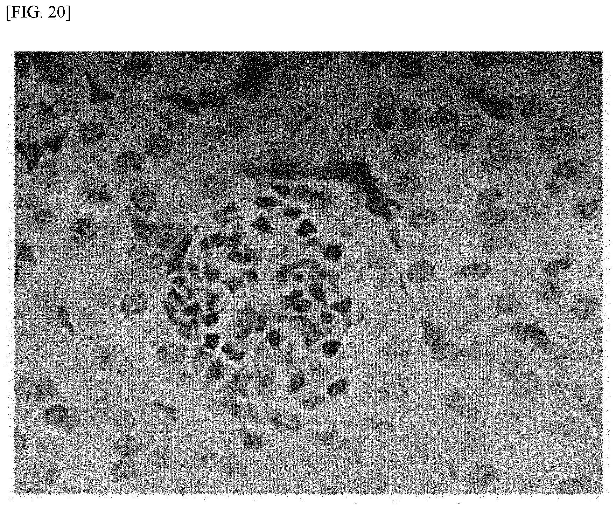

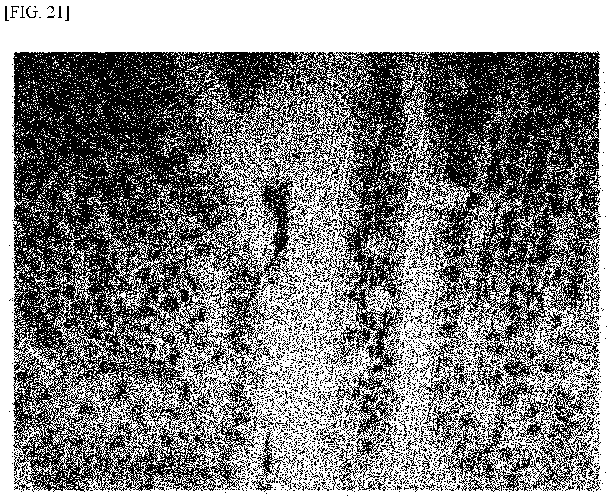

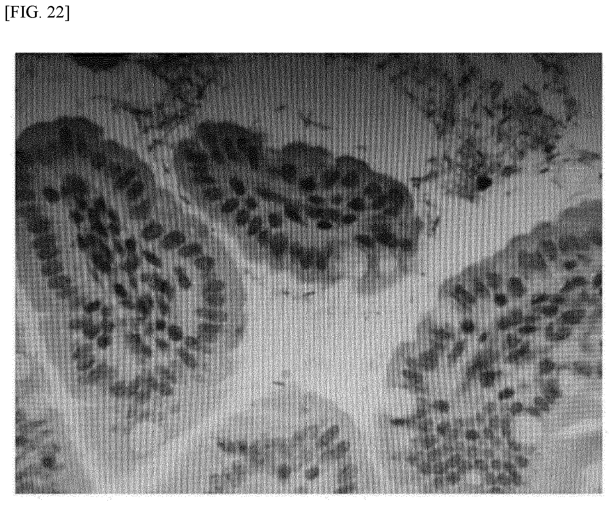

As illustrated in FIGS. 17 to 22, it was identified that in hepatocytes, kidney cells, and ileal villous cells, the cytoplasm was stained brown.

From the above results, it can be seen that by using the method for producing an animal model according to the present invention, it is possible to very efficiently produce an animal model that can be effectively used clinically without limitation resulting from species specificity, because a human-derived viral lipid layer is replaced with a target animal model-derived lipid layer.

Although specific parts of the present invention have been described in detail, it will be apparent to those skilled in the art that these specific descriptions are provided only for preferred embodiments and the scope of the present invention is not limited thereby. Therefore, the substantial scope of the present invention should be defined by the appended claims and equivalents thereof.

### Industrial applicability

According to the present invention, for a human-derived lipid layer of a virus, a bile acid or a bile acid derivative allows the lipid layer to be replaced with a target animal model-derived lipid layer; and this makes it possible to produce virus-infected cell line or animal model which can be effectively used clinically without limitation resulting from species specificity. Accordingly, there is industrial applicability in that the cell line and the animal model can be used to very effectively screen a therapeutic agent for a disease caused by a virus including a lipid bilayer envelope, thereby remarkably decreasing social costs associated therewith.

## Claims

1. A method for producing a virus-infected cell line, comprising steps of:
mixing an enveloped virus including a lipid bilayer with a bile acid or a bile acid derivative; and
adding, to a culture medium of a cell line, the virus mixed with the bile acid or the bile acid derivative.

2. The method according to claim 1, wherein the method for producing a virus-infected cell line is performed *in vitro.*

3. The method according to claim 1, wherein the enveloped virus including a lipid bilayer is a hepatitis virus.

4. The method according to claim 3, wherein the hepatitis virus is hepatitis B virus (HBV) or hepatitis C virus (HCV).

5. The method according to claim 1, wherein the bile acid derivative is any one or more selected from the group consisting of chenodeoxycholic acid, deoxycholic acid, lithocholic acid, taurocholic acid, glycocholic acid, taurochenodeoxycholic acid, glycochenodeoxycholic acid, glycodeoxycholic acid, taurodeoxycholic acid, glycolithocholic acid, taurolithocholic acid, and muricholic acid.

6. The method according to claim 1, wherein the cell line is any one or more selected from the group consisting of a liver cell line, a lung cell line, a kidney cell line, a muscle cell line, a breast cell line, an immune T cell line, and a reproductive cell line.

7. The method according to claim 6, wherein the cell line is derived from humans or mice.

8. The method according to claim 7, wherein the human-derived cell line is HepG2.

9. A virus-infected cell line, produced by mixing an enveloped virus including a lipid bilayer with a bile acid or a bile acid derivative, and adding, to a culture medium of a cell line, the virus mixed with the bile acid or the bile acid derivative.

10. The cell line according to claim 9, wherein the enveloped virus is a hepatitis virus.

11. A method for producing a virus-infected animal model, comprising steps of:
preparing a target animal other than humans;
causing a lipid layer of an enveloped virus including a lipid bilayer to be replaced with a lipid layer derived from the target animal; and
injecting, into the target animal, the virus for which the lipid layer replacement has been achieved.

12. The method according to claim 11, wherein the step of causing a lipid layer of an enveloped virus including a lipid bilayer to be replaced with a lipid layer derived from the target animal is performed through steps of:
mixing the enveloped virus including a lipid layer with a bile acid or a bile acid derivative; and
adding the virus mixed with the bile acid or the bile acid derivative to a culture medium of a cell line derived from the target animal.

13. The method according to claim 11, wherein the target animal is a vertebrate animal other than humans.

14. The method according to claim 13, wherein the vertebrate animal is any one or more selected from the group consisting of mice, rats, rabbits, birds, cats, dogs, pigs, sheep, goats, deer, horses, and cattle.

15. The method according to claim 11, wherein the enveloped virus including a lipid layer is a hepatitis virus.

16. The method according to claim 15, wherein the hepatitis virus is hepatitis B virus (HBV) or hepatitis C virus (HCV).

17. The method according to claim 12, wherein the bile acid derivative is any one or more selected from the group consisting of chenodeoxycholic acid, deoxycholic acid, lithocholic acid, taurocholic acid, glycocholic acid, taurochenodeoxycholic acid, glycochenodeoxycholic acid, glycodeoxycholic acid, taurodeoxycholic acid, glycolithocholic acid, taurolithocholic acid, and muricholic acid.

18. A virus-infected animal model, produced by preparing a target animal other than humans; causing a lipid bilayer envelope of an enveloped virus containing the lipid bilayer to be replaced with a lipid bilayer envelope derived from the target animal; and injecting, into the target animal, the virus for which the lipid bilayer envelope replacement has been achieved.

19. The method according to claim 18, wherein the enveloped virus including a lipid bilayer envelope is a hepatitis virus.

20. A method for screening a therapeutic candidate for a viral disease, comprising steps of:
culturing the virus-infected cell line according to claim 9; and
adding, to the cell line, a therapeutic candidate for a viral disease.

21. The method according to claim 20, wherein the viral disease is viral hepatitis.

22. The method according to claim 20, further comprising:
a step of identifying presence or absence of a viral genome or antigen, in each of the cultured virus-infected cell line, and the virus-infected cell line to which the therapeutic candidate has been added.

23. The method according to claim 22, wherein in a case where a level of the viral genome or surface antigen present is decreased in the virus-infected cell line, to which the therapeutic candidate has been added, as compared with the cultured virus-infected cell line, the candidate is selected as a therapeutic substance for the viral disease.

24. A method for screening a therapeutic candidate for a viral disease, comprising steps of:
rearing the virus-infected animal model according to claim 18; and
adding, to the virus-infected animal model, a therapeutic candidate for a viral disease.

25. The method according to claim 24, wherein the viral disease is viral hepatitis.

26. The method according to claim 24, further comprising:
a step of identifying presence or absence of a viral genome or antigen, in each of the reared virus-infected animal model, and the virus-infected animal model to which the therapeutic candidate has been added.

27. The method according to claim 26, wherein in a case where a level of the viral genome or surface antigen present is decreased in the virus-infected animal model, to which the therapeutic candidate has been added, as compared with the reared virus-infected animal model, the candidate is selected as a therapeutic substance for the viral disease.
